(19) **Europäisches Patentamt**
**European Patent Office**
**Office européen des brevets**

(11) **EP 2 233 923 B1**

(12) **FASCICULE DE BREVET EUROPEEN**

(45) Date de publication et mention
de la délivrance du brevet:
**22.08.2012 Bulletin 2012/34**

(51) Int Cl.:
***G01N 33/49*** *(2006.01)*

(21) Numéro de dépôt: **10290082.6**

(22) Date de dépôt: **18.02.2010**

(54) **Procédé et dispositif pour la caractérisation de la dynamique de coagulation ou sédimentation d'un fluide tel que le sang ou le plasma sanguin**

Methode und Vorrichtung zur Charakterisierung der Gerinnungssdynamik or Sedimentation von Blut oder Blutplasma

Method and apparatus for the characterisation of the coagulation dynamics or sedimentation of blood or blood serum

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **26.03.2009 FR 0901435**

(43) Date de publication de la demande:
**29.09.2010 Bulletin 2010/39**

(73) Titulaire: **Commissariat à l'Énergie Atomique et aux Énergies Alternatives 75015 Paris (FR)**

(72) Inventeurs:
• **Pouteau, Patrick 38240 Meylan (FR)**
• **Faivre, Magali 38000 Grenoble (FR)**
• **Peltie, Philippe 38760 Saint Paul de Varces (FR)**
• **Planat Chretien, Anne 38120 St. Egreve (FR)**

(74) Mandataire: **Priori, Enrico et al Cabinet Orès 36, rue de St. Pétersbourg 75008 Paris (FR)**

(56) Documents cités:
**US-A- 4 756 884**

• **PIEDERRIERE, Y.: "ETUDE DU SPECKLE DE MILIEUX DIFFUSANTS LIQUIDES. APPLICATION A LA DETERMINATION DE PARAMETRES BIOPHYSIQUES." 15 décembre 2003 (2003-12-15), L'UNIVERSITÉ DE BRETAGNE OCCIDENTALE, U.F.R. SCIENCES ET TECHNIQUES DE BREST , Brest , XP002591394 * Sections 2.3, 2.4.3, 4.4 * * ***
• **PIEDERRIÈRE, Y; CARIOU, J; GUERN, Y; LE BRUN, GUY; LE JEUNE, B; LOTRIAN, J: "Evaluation of blood plasma coagulation dynamics by speckle analysis" J. BIOMED. OPT., vol. 9, no. 2, 22 mars 2004 (2004-03-22), pages 408-412, XP002551759**
• **PIEDERRIERE Y; BOULVENT F; LE BRUN G; LE JEUNE B; GUERN Y ; CARIOU J;: "Speckle and polarization for biomedical applications" PROC. OF SPIE, vol. 6341, 13 septembre 2006 (2006-09-13), pages 634106.1-634106.5, XP002551830**
• **KALCHENKO, V; BRILL, A; BAYEWITCH, M; FINE, I; ZHAROV, V; GALANZHA, E; TUCHIN, V; HARMELIN, A: "In vivo dynamic light scattering imaging of blood coagulation" J. BIOMED. OPT., vol. 12, no. 5, 12 septembre 2007 (2007-09-12), pages 052002-1-052002-4, XP002551829**
• **DAN CHICEA: "Results of sediment motion visualization by a modified LASCA technique", PROCEEDINGS OF SPIE, SPIE, USA, vol. 6785, 1 January 2007 (2007-01-01), pages 67851O-1, XP007918645, ISSN: 0277-786X, DOI: DOI: 10.1117/12.757883**

• SEEMANTINI K NADKARNI ET AL: "Characterization of Atherosclerotic Plaques by Laser Speckle Imaging", CIRCULATION, LIPPINCOTT WILLIAMS & WILKINS, US, vol. 112, 1 January 2005 (2005-01-01), pages 885-892, XP007918646, ISSN: 0009-7322, DOI: DOI: 10.1161/CIRCULATIONAHA.104.520098 [retrieved on 2005-08-01]

• RAJAN ET AL: "Speckle size and decorrelation time; space-time correlation analysis of coherent light dynamically scattered from turbid media", OPTICS COMMUNICATIONS, NORTH-HOLLAND PUBLISHING CO. AMSTERDAM, NL, vol. 281, no. 6, 21 December 2007 (2007-12-21), pages 1755-1760, XP022442919, ISSN: 0030-4018, DOI: DOI:10.1016/J.OPTCOM.2007.11.025

## Description

L'invention porte sur un procédé de caractérisation de la dynamique de coagulation ou de sédimentation d'un fluide tel que le sang entier, une fraction du sang ou le plasma sanguin, ainsi que sur un dispositif pour la mise en oeuvre d'un tel procédé.

La mesure du temps de coagulation du sang présente un grand intérêt médical. En effet, les troubles de la coagulation peuvent entrainer des perturbations physiologiques importantes telles que des saignements abondants lors d'interventions médicales ou de blessures hémorragiques (temps trop long) ou telles que des thromboses ou embolies (temps trop court). De très nombreuses maladies, pathologies, traitements médicaux, suivis thérapeutiques nécessitent un contrôle plus ou moins régulier de ce temps de coagulation. Bien souvent cela se traduit par un prélèvement veineux nécessitant l'intervention d'un spécialiste. Ce prélèvement est ensuite analysé dans un laboratoire spécialisé, où plusieurs tests différents peuvent être réalisés pour mesurer le temps de coagulation, en fonction de la pathologie ou du traitement thérapeutique du patient qui est l'objet de l'analyse. On peut citer, en particulier: le temps de Quick (Facteur TQ), le temps de céphaline activée (Facteur TCA), le temps de céphaline kaolin (TCK). Souvent, ce ne sont pas directement les valeurs absolues de ces temps de coagulation qui sont intéressantes pour le cliniciens, mais leurs rapports avec des temps témoins des mêmes tests, réalisés sur un échantillon témoin constitué à partir d'un pool (ou mélange) d'une cinquantaine d'échantillons de patients réputés normaux. En outre, le temps de coagulation mesuré dépend de la méthode physique de caractérisation du phénomène de coagulation, de la façon de mélanger l'échantillon avec le facteur de coagulation étudié (temps de mélange) ainsi que des réactifs utilisés pour déclencher la réaction. Il est donc usuel d'appliquer une correction afin d'obtenir un résultat indépendant de ces facteurs. On peut citer, à titre d'exemple, l'INR (« International Normalized Ratio », c'est à dire rapport international normalisé) calculé à partir du temps de Quick divisé par le temps témoin et élevé à la puissance ISI (indice de sensibilité international) qui est donné par le fabricant de lot de réactifs utilisé pour la mise en oeuvre du test.

Classiquement, la mesure du temps de coagulation sanguine comporte les étapes suivantes.

Dans un premier temps le sang est prélevé du patient dans un tube contenant un anticoagulant adapté permettant un délai raisonnable de transport entre le lieu de prélèvement et le lieu d'analyse (au minimum plusieurs minutes) sans que l'échantillon ne coagule.

Ensuite, le plasma est extrait de l'échantillon de sang par centrifugation et mélangé dans les bonnes proportions avec différents réactifs nécessaires à l'inhibition de l'anticoagulant utilisé lors du prélèvement (par exemple, des ions calcium) et avec les réactifs nécessaires au déclenchement de la coagulation (par exemple, de la thromboplastine) suivant le facteur étudié.

Enfin, la mesure du temps de coagulation proprement dite est effectuée à l'aide de différents équipements.

Le document US 4,252,536 décrit un dispositif et un procédé optique de mesure du temps de coagulation. Ce dispositif et ce procédé se basent sur l'évolution de l'intensité d'un signal optique détecté du fait de la modification de la diffusion dans un échantillon de plasma lors de la formation d'un caillot de sang coagulé.

Le document US 3,635,678 décrit une autre méthode couramment utilisée, consistant à introduire dans l'échantillon de plasma une bille magnétique, mise en mouvement oscillatoire à l'aide d'un aimant ou électroaimant extérieur. L'observation du déplacement de la bille est réalisée par voie optique. La mesure du temps au bout duquel la bille se fige dans le plasma, immobilisée par le caillot qui se forme, correspond au temps de coagulation.

Ces méthodes ne conviennent pas à l'utilisation avec du sang entier, qui est trop opaque.

Le document US 6,352,630 décrit un procédé électrochimique de mesure du temps de coagulation. La mise en oeuvre de ce procédé nécessite un consommable comportant des électrodes en contact avec l'échantillon biologique, un appareillage permettant la reprise de contacts électriques sur le consommable, et l'ajout audit échantillon d'agents électrochimiques permettant la mesure.

L'article de Yann Piederrière et al. « Evaluation of blood plasma coagulation dynamics by speckle analysis » décrit deux procédés d'étude de la dynamique de coagulation du sang par analyse de la granularité (ou des tavelures) laser.

Ces procédés se basent sur l'observation du fait que les particules (plaquettes, protéines) en suspension dans le plasma diffractent et diffusent la lumière ; si un échantillon de plasma est éclairé par un faisceau laser, spatialement et temporellement cohérent, il en résulte un motif de granularité (ou de tavelures, « speckle » en anglais). A cause du mouvement brownien des particules, ce motif varie dans le temps, tant que le plasma reste liquide, puis se fige une fois le caillot formé.

Dans le premier procédé décrit dans l'article précité, l'intensité lumineuse en correspondance d'un point du motif de granularité est enregistrée en fonction du temps. La mise en oeuvre de ce procédé est assez délicate : en effet, la transparence du plasma diminue au cours du processus de coagulation ; il est donc nécessaire d'incrémenter l'intensité lumineuse d'éclairage au cours du temps, ou d'éclairer l'échantillon assez fortement pendant toute la durée d'acquisition, au risque de saturer le détecteur. En outre, comme seule l'intensité lumineuse en un point est considérée, le signal optique est faible - ce qui implique un rapport signal sur bruit insatisfaisant - à moins d'utiliser une intensité lumineuse d'éclairage relativement importante.

Le deuxième procédé décrit comporte l'acquisition d'une série temporelle d'images du motif de granularité, et la détermination du contraste de chaque image. Avant la formation du caillot, le mouvement brownien des particules en suspension « brouille » les images, et réduit leur contraste ; ce dernier augmente lorsque le motif de granularité se fige à cause de la coagulation du plasma. De l'aveu même des auteurs, cette méthode ne permet pas une détermination précise du temps de coagulation.

Tous les procédés décrits ci-dessus utilisent du plasma, et ne peuvent pas fonctionner avec du sang entier. Ils nécessitent donc d'une étape préalable de fractionnement du sang, qui ne peut être effectuée que dans un laboratoire spécialisé.

L'invention vise à surmonter au moins certains des inconvénients de l'art antérieur. En particulier, le dispositif et le procédé de l'invention permettent l'étude de la dynamique de coagulation tant du sang entier que du plasma. En outre, ils sont simples et économiques à mettre en oeuvre, sur le lieu de traitement, et fournissent des résultats précis.

Par ailleurs, l'invention permet également d'étudier la dynamique de sédimentation du sang, qui est également d'intérêt médical.

Un objet de l'invention est un Procédé de caractérisation de la dynamique de coagulation ou de sédimentation d'un fluide tel que le sang entier, une fraction du sang ou le plasma sanguin, comportant :

- l'éclairage d'un échantillon dudit fluide (ES) avec un faisceau de lumière cohérente ;
- l'acquisition d'une série temporelle d'images d'un motif de granularité engendré par l'interaction entre ledit échantillon et ledit faisceau de lumière spatialement cohérente ; et
- le traitement de ladite série temporelle d'images ;

**caractérisé en ce que** ladite étape de traitement comporte :

- le calcul d'une fonction (r) représentative de la variation spatiale dudit motif de granularité entre deux ou plusieurs images de la série ; et
- l'analyse de l'évolution temporelle de ladite fonction.

Le procédé de l'invention se différencie de celui connu de l'article précité d'Yann Piederrière et al. essentiellement par le traitement des images qu'il met en oeuvre. Dans le procédé de l'art antérieur, chaque image de granularité est considérée indépendamment des autres, et traitée de manière à fournir une valeur numérique globale (le contraste), indicative de la fluidité de l'échantillon ; la variation temporelle de ces valeurs numériques fournit l'information requise sur la dynamique de coagulation. Au contraire, dans le cas de l'invention, deux images successives, acquises à des instants différents, sont analysées conjointement, pixel par pixel ; c'est seulement après cette analyse conjointe que l'on calcule une fonction, ou valeur numérique, « globale », indicative du changement du motif de granularité entre deux temps d'acquisition d'images. Une analyse de l'évolution temporelle de cette fonction fournit une information sur la dynamique de coagulation ou de sédimentation du fluide.

Les expériences réalisées par les inventeurs montrent que la méthode de traitement des données mise en oeuvre par l'invention fournit des résultats plus précis que celle de l'art antérieur. En outre, contrairement à cette dernière, elle convient également à l'étude de la coagulation du sang entier, et pas seulement du plasma (bien que les raisons de cela ne soient pas bien comprises).

Un autre objet de l'invention est un dispositif pour la mise en oeuvre d'un procédé de caractérisation de la dynamique de coagulation ou de sédimentation d'un fluide tel que décrit ci-dessus, comportant :

- un dispositif microfluidique présentant une veine fluidique susceptible de recevoir un échantillon dudit fluide et permettant son éclairage ;
- un moyen d'éclairage dudit échantillon par un faisceau de lumière cohérente ;
- un capteur d'images agencé pour permettre l'acquisition d'une série temporelle d'images d'un motif optique de granularité engendré par l'interaction entre ledit échantillon et ledit faisceau de lumière cohérente ; et
- un moyen de traitement de ladite série temporelle d'images ;

**caractérisé en ce que** ledit moyen de traitement est adapté pour calculer une fonction représentative de la variation dudit motif de granularité entre deux ou plusieurs images de la série et pour analyser l'évolution temporelle de ladite fonction.

Différents modes de réalisation du procédé et du dispositif de l'invention font l'objet des revendications dépendantes.

D'autres caractéristiques, détails et avantages de l'invention ressortiront à la lecture de la description faite en référence aux dessins annexés donnés à titre d'exemple et qui représentent, respectivement :

- La figure 1, une image de granularité d'un échantillon de sang complet ;
- Les figures 2A et 2B, une chambre microfluidique utilisée comme porte-échantillon ;
- Les figures 3A et 3B, des schémas correspondant à deux modes de réalisation d'un dispositif selon l'invention ;
- Les figures 4A et 4B, respectivement, une courbe de corrélation obtenue par un procédé selon l'invention et opérant sur du sang entier, et sa dérivée ;
- Les figures 5A et 5B, respectivement, une courbe de corrélation obtenue par un procédé selon l'invention et opérant sur du plasma, et sa dérivée ;
- Les figures 6A et 6B, des graphiques permettant de comparer les résultats obtenus en appliquant la technique de l'invention à ceux fournis par une méthode conventionnelle de mesure du temps de Quick (6A) et de l'INR (6B) ;
- La figure 7, un graphique montrant la faible influence de l'hématocrite sur les résultats obtenus en appliquant la technique de l'invention ;
- La figure 8, un graphique montrant la variation de corrélation obtenue par un procédé selon un mode de réalisation de l'invention à travers un échantillon de sang et provoqué par la sédimentation des cellules ; et
- Les figures 9A, 9B, 9C et 10A, 10B, 10C, des graphiques permettant de comparer le procédé de l'invention et celui décrit par l'article précité de Yann Piederrière et al.

Comme cela est bien connu en optique, un objet diffusant ou une surface rugueuse soumis à un rayonnement laser (ou plus généralement à un rayonnement spatialement et temporellement cohérent) forme une image granulaire, à cause des effets de diffraction engendrés par la rugosité ou les centres de diffusion, et des interférences entre les motifs de diffraction individuels. Cet effet est connu sous le nom de granularité ou tavelures, ou « speckle » en anglais.

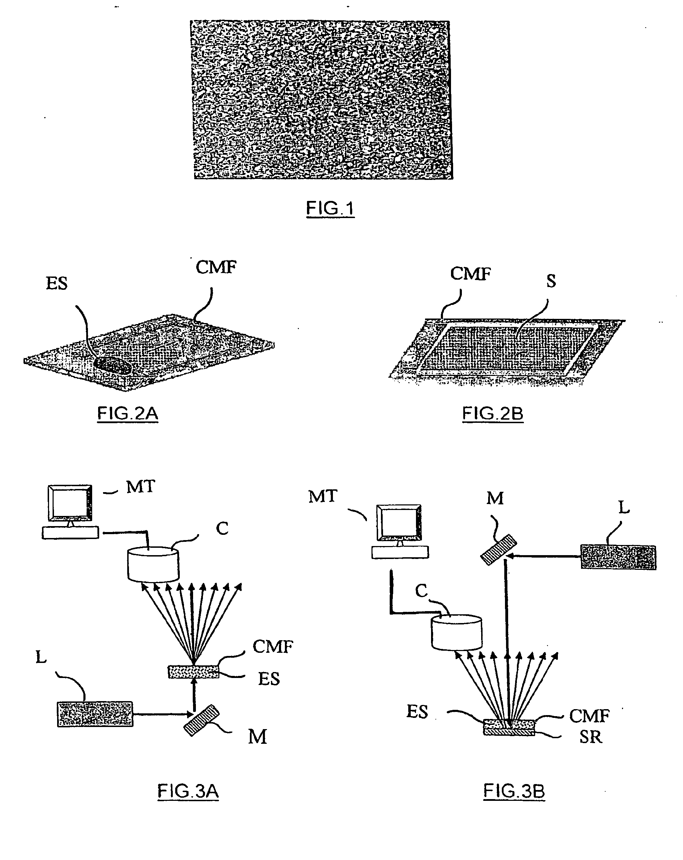

La figure 1 représente l'image de granularité d'un échantillon de sang total, dans lequel les cellules (globules blancs et rouges) et les autres particules en suspension dans le plasma (plaquettes, grosses protéines) agissent comme centres de diffusion de la lumière.

Les particules en suspension dans un échantillon sanguin sont animées d'un mouvement incessant ; l'image de granularité évolue donc au cours du temps, jusqu'à se figer quand le sang est complètement coagulé et forme un caillot solide. C'est cet effet qui, conformément à l'invention, permet la mesure du temps de coagulation.

Comme le montrent les figures 2A à 3B, un dispositif pour la mise en oeuvre du procédé de l'invention comporte une chambre microfluidique CMF, au moins partiellement transparente, une source d'éclairage en lumière spatialement et temporellement cohérente, telle qu'un laser semiconducteur ou à Hélium-Néon L, et une caméra C. La référence M indique un miroir utilisé pour diriger le faisceau laser vers la chambre fluidique.

Selon le mode de réalisation exemplaire de l'invention, la chambre microfluidique utilisée est composée de 2 lames de verre séparées par des espaceurs et maintenues à une distance réciproque de $160\mu$m. Elle est préalablement placée sur le banc de mesure.

Une quantité de $10\mu$L de sang total est ajoutée à $20\mu$L de Néoplastine® (mélange de thromboplastine et de chlorure de Calcium, fabriqué par STAGO diagnostics).

La solution est homogénéisée par aspirations et refoulement successifs dans une micropipette de $20\mu$L de ce mélange sont déposés à l'entrée de la chambre microfluidique qui se remplit par simple migration capillaire comme représenté sur les figures 2A et 2B, où la référence ES indique l'échantillon constitué par le sang et les réactifs.

La mesure est déclenchée au moment où les réactifs sont mis en présence. Il existe donc un délai, typiquement d'une dizaine ou de quelques dizaines de secondes, entre le début de la mesure et le moment où l'on pourra visualiser effectivement des images qui correspondent au remplissage de la chambre, puis à la coagulation. L'instant initial de mesure est pris au moment du mélange.

Les dimensions du dispositif microfluidique CMF et les volumes de sang et de réactifs donnés ci-dessus ne sont qu'indicatifs, et ne constituent nullement des limitations de l'invention. Plus généralement, dans le cas du sang entier, la veine microfluidique définie par la chambre CMF pourra avoir une épaisseur allant de quelques dizaines de micromètres à 1 mm environ. Si l'épaisseur est trop faible, il n'y aura pas suffisamment de particules sur le trajet du faisceau lumineux, et le contraste de l'image de granularité sera insuffisant ; si l'épaisseur est trop importante, l'absorption de la lumière par le sang donnera une image trop sombre pour être exploitable.

La figure 3A illustre schématiquement un premier mode de réalisation de l'invention, dans lequel la chambre microfluidique CMF est entièrement transparente, constituée par exemple par deux lames de verre, et la mesure est effectuée par transmission. Pour ce faire, le faisceau lumineux d'éclairage en lumière cohérente est incident sur la chambre CMF d'un premier côté de cette dernière, et la caméra C est agencée du côté opposé. On remarquera que la caméra C est décalée par rapport au trajet du faisceau lumineux en l'absence de diffusion/diffraction, afin d'éviter son éblouissement.

Comme cela a été évoqué plus haut, la source de lumière L peut typiquement être un laser à Hélium-Néon ou à semi-conducteur. La longueur d'onde du rayonnement est avantageusement d'environ 650 - 690 nm, afin de permettre une bonne pénétration dans le sang. Une puissance d'éclairage comprise entre 100 $\mu$W et quelques milliwatts, ou dizaines de milliwatts, peut être suffisante.

L'épaisseur de la chambre microfluidique CMF peut être généralement comprise entre 20 et 1000 $\mu$m, et de préférence entre 30 $\mu$m et 300 $\mu$m.

La surface du faisceau lumineux incident à la chambre microfluidique peut être comprise de préférence entre 10 $\mu$m$^2$ environ et quelques mm$^2$, les dimensions de la chambre microfluidique étant ajustées en conséquence.

La caméra C est essentiellement constituée d'un capteur d'images, de type matrice CCD ou capteur d'image en technologie CMOS, disposé à une distance de quelques centimètres ou dizaines de centimètres de la chambre microfluidique (une telle distance de propagation est nécessaire à la formation d'une image de granularité), sans système de focalisation. L'échantillonnage peut être modeste : un capteur de 30 x 30 pixels a été utilisé avec des résultats satisfaisants. Concrètement, il peut s'agir d'une caméra de type « webcam » privée de sa lentille, voire du système optique d'une souris à laser d'ordinateur.

La cadence d'acquisition des images n'a pas à être particulièrement élevée : une cadence de 0,5 Hz s'est avérée suffisante pour étudier la dynamique de coagulation du sang. Une cadence trop élevée est inutile : l'intervalle temporel séparant deux images successives doit être au moins égale au temps caractéristique de variation de l'image de granularité.

Le temps d'acquisition de chaque image peut être avantageusement de 50 ms, voire moins si l'intensité lumineuse est suffisante. Un temps d'acquisition trop long conduit à une diminution du contraste des tavelures, car le mouvement des particules en suspension peut alors être suffisamment rapide pour rendre l'image floue.

La figure 3B illustre schématiquement un deuxième mode de réalisation de l'invention, dans lequel la chambre microfluidique CMF constituée par une lame supérieure transparente et un substrat réfléchissant SR, par exemple en silicium. Dans ce cas, le faisceau laser traverse la lame transparente et l'échantillon, puis est partiellement réfléchi par le substrat SR, traverse à nouveau l'échantillon et le signal est ensuite détecté par la caméra C placée du même côté de l'échantillon que le laser L. Cette configuration peut s'avérer intéressante si, par exemple, l'échantillon à besoin d'être agité ou chauffé par un module placé au-dessous de la chambre microfluidique. Elle peut également permettre l'intégration d'un dispositif selon l'invention à une puce microfluidique réalisée en silicium, plastique, verre. Une telle puce microfluidique peut comporter des moyens d'amenée, une zone de mélange avec des réactifs, liquides ou secs, préalablement disposés dans la chambre microfluidique, une zone permettant une homogénéisation du mélange, par des perturbations éventuellement externes (agitateur acoustique), suivie d'une zone d'analyse. Les réactifs utilisés peuvent être secs, par exemple lyophilisés. La zone d'analyse peut-également être placée au niveau de la zone de mélange avec des réactifs secs. Le moyen d'amenée peut directement être une aiguille de prélèvement, l'aspiration du sang étant alors réalisée soit par capillarité, soit par aspiration. Des moyens de chauffage et de contrôle de la température peuvent être rapportés au contact ou à proximité de la chambre microfluidique.

Dans ce deuxième mode de réalisation de l'invention, l'épaisseur de la veine microfluidique pourra être la moitié que celle du premier mode de réalisation, de manière à donner un chemin optique dans l'échantillon ayant une même longueur.

Quel que soit le mode de réalisation retenu, les images acquises par la caméra C sont numérisées et transmises, en différé ou en temps réel, à un moyen de traitement des données MT, qui peut être typiquement un ordinateur portable ou de bureau équipé d'un logiciel approprié, ou un composant de type DSP (processeur numérique de signaux) permettant de traiter directement les images, les moyens de traitement pouvant également être directement intégrés dans la puce Silicium de prise d'images.

Comme discuté plus haut, l'étape essentielle du traitement des images selon l'invention est représentée par le calcul d'une fonction représentative de la variation spatiale dudit motif de granularité entre deux ou plusieurs images acquises à des instants différents.

Plusieurs fonctions de ce type peuvent être envisagées.

Une première possibilité consiste à calculer, pixel par pixel, la différence d'intensité entre deux images successives, et à sommer les valeurs absolues de ces différences entre deux images pour donner une valeur de la variation entre deux images successives :

$$f_1 = \sum_{i=1}^{N} \left| x_i - y_i \right|$$

où :

- N est le nombre total de pixels ;
- $x_i$ (avec i=1 - N) sont les valeurs d'intensité des pixels d'une image ;
- $y_i$ sont les valeurs d'intensité des pixels de l'image précédent ou suivant x.

Selon une variante, on peut calculer

$$f_1 = \sum_{i=1}^{N} |x_i - y_i|^2$$

Une deuxième possibilité consiste à réaliser un calcul du facteur de corrélation r entre deux images successives pour donner une valeur de la variation entre ces deux images :

$$f_2 = r = \frac{\sum_{i=1}^{N} \sqrt{(x_i - \bar{x})^2} \times \sqrt{(y_i - \bar{y})^2}}{\sqrt{\sum_{i=1}^{N} (x_i - \bar{x})^2} \times \sqrt{\sum_{i=1}^{N} (y_i - \bar{y})^2}}$$

où :

-    N est le nombre total de pixels ;
-    $x_i$ (avec i=1 - N) sont les valeurs d'intensité des pixels d'une image ;
-    $\bar{x}$ est la valeur moyenne d'intensité pour l'image x :

$$\bar{x} = \frac{1}{N} \sum_{i=1}^{N} x_i \; ;$$

-    $y_i$ sont les valeurs d'intensité des pixels de l'image précédent ou suivant x ; et
-    $\bar{y}$ est la valeur moyenne d'intensité pour l'image y :

$$\bar{y} = \frac{1}{N} \sum_{i=1}^{N} y_i .$$

Le dénominateur utilisé dans le calcul de la fonction $f_2$ est optionnel. Il est néanmoins utile en ce qu'il permet de neutraliser l'effet des variations de luminosité des images.

D'une façon plus générale, on pourra utiliser toute fonction permettant de mesurer le degré de similitude entre deux images.

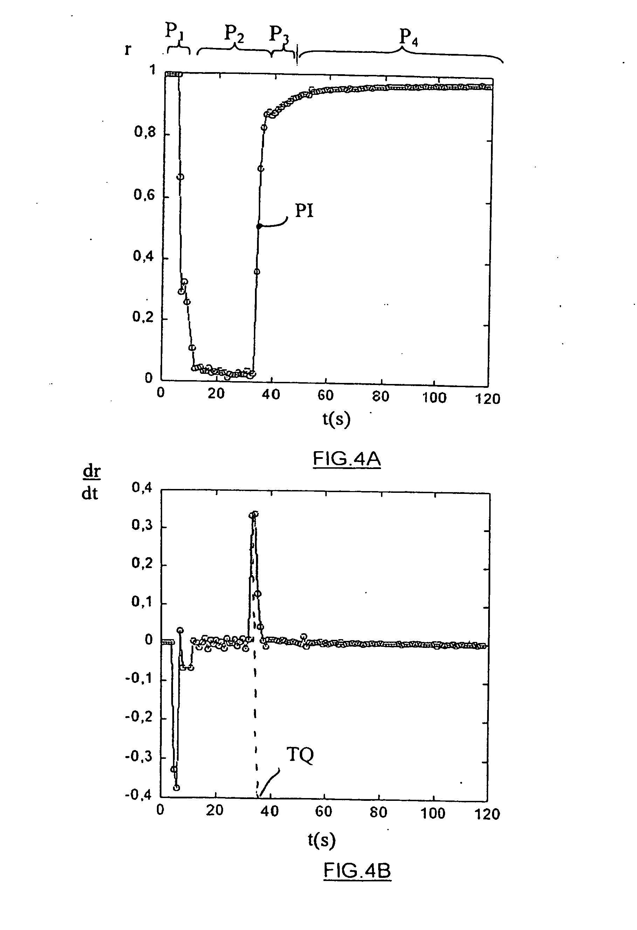

La figure 4A montre le graphique d'une courbe représentant la valeur du coefficient de corrélation r en fonction du temps t (en secondes), obtenue dans les conditions expérimentales décrites ci-dessus. La cadence d'acquisition est de 1 Hz, et le temps d'acquisition de 50 ms.

Sur la courbe, on peut distinguer quatre phases.

Initialement (phase $P_1$) la chambre microfluidique CMF est vide. Il n'y a pas de variation de la granularité et le facteur de corrélation r est proche de 1 (il vaudrait exactement 1 s'il n'y avait pas de bruit, tant électronique qu'optique).

Ensuite, le sang est introduit dans la chambre microfluidique, et le facteur de corrélation décroît brusquement.

Pendant la deuxième phase $P_2$, précédant la coagulation proprement dite, le facteur de corrélation reste très bas. En effet, entre une image et la successive, le motif de granularité change sensiblement à cause du mouvement des particules en suspension dans le plasma sanguin. Il est intéressant de rappeler que, pendant cette phase, se produit une cascade de réactions préparant la coagulation.

La coagulation proprement dite, au cours de laquelle les cellules de sang ralentissent voire cessent leur déplacement, se produit au cours de la troisième phase, P3, qui voit le facteur de corrélation augmenter rapidement. Lorsque le facteur de coagulation utilisé est la thromboplastine, on parle de temps de Quick.

Le temps de coagulation TC est défini, de manière conventionnelle, comme l'instant pour lequel la courbe r(t) présente un point d'inflexion PI (le temps de Quick TQ correspond au temps de coagulation TC lorsque le facteur coagulant est la Thromboplastine). Pendant la quatrième et dernière phase P4, le coefficient de corrélation r n'évolue pratiquement plus, indiquant qu'un caillot s'est formé figeant les particules en suspension et, par conséquent, le motif de granularité. En fait, le sérum encore présent permet une certaine agitation des cellules, c'est pourquoi le coefficient de corrélation n'est pas strictement égal à 1.

Le point d'inflexion PI peut être déterminé en identifiant un maximum local de la dérivée temporelle de r(t), comme illustré par la figure 4B.

Le procédé de l'invention a été répété 20 fois, avec du sang d'un même donneur sain, pour tester sa répétabilité. Le temps de coagulation moyen mesuré était de 34s avec un écart-type de 1,5s correspondant à un coefficient de variation de 4,5%, ce qui est très satisfaisant compte tenu du fait que le déclenchement de la mesure était effectué manuellement.

Les performances de la technique ont été évaluées sur une gamme de mesure représentative de la réalité de la variabilité entre individus, ces derniers rassemblant des donneurs sains et des donneurs pathologiques Cette évaluation, effectuée sur une base de 66 patients, s'est déroulée dans les locaux des Hospices Civils de Lyon (HCL). La technique de mesure de l'invention a été confrontée à une technique classique, par mesure de la transmission optique du plasma, mise en oeuvre dans ce laboratoire d'analyse hospitalier.

Le tableau suivant résume les caractéristiques des 2 techniques :

| | Référence (HCL) | Invention |
|---|---|---|
| Système de mesure | Automate MDA, société Trinity Biotech : mesure de transmittance optique | Corrélation d'images de granulosité laser |
| Echantillon | 10 $\mu$l de plasma prélevé dans un tube de 5 ml de sang citraté centrifugé | 10 $\mu$l de sang total citraté |
| Température | 37 °C | Ambiante |
| Réplique des mesures | 1 | 3 |
| Plage temporelle | 10 s - 60 s | 25 s - 150 s |
| Durée de l'analyse | 20 min de centrifugation + 20 min dans l'automate = 40 min | 7 min |
| Coefficient de variation | <5% | <10% |

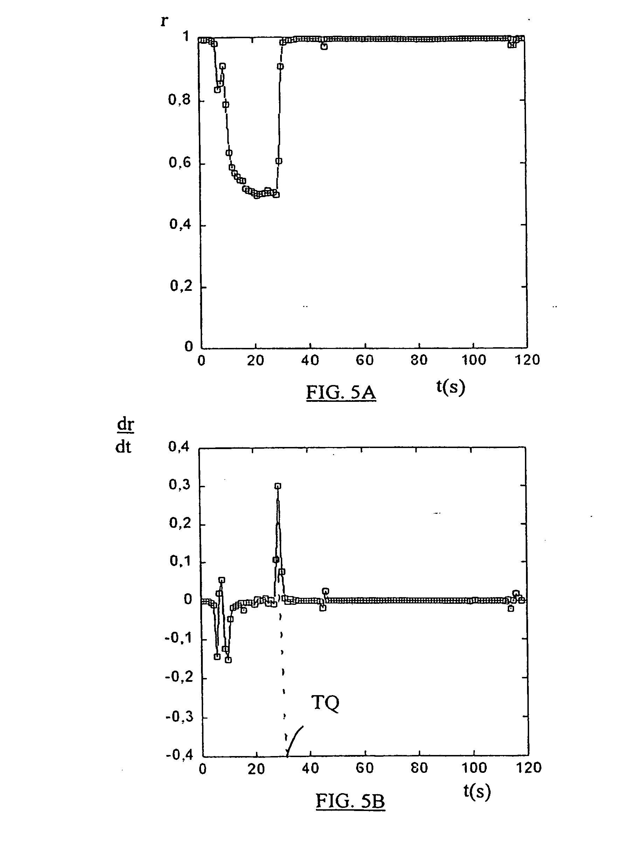

Les figures 5A et 5B correspondent aux figures 4A et 4B, respectivement, mais se rapportent à une mesure effectuée pour du plasma sanguin. L'épaisseur de la veine fluidique est de 50 $\mu$m dans les deux cas. On peut constater que la méthode de l'invention donne des résultats satisfaisants même dans ce cas ; cependant, la variation du coefficient de corrélation est moindre à cause de la densité de diffuseurs qui est beaucoup plus faible dans le plasma (il s'agit dans ce cas essentiellement de plaquettes et de protéines) que dans le sang entier (où la contribution des cellules est dominante).

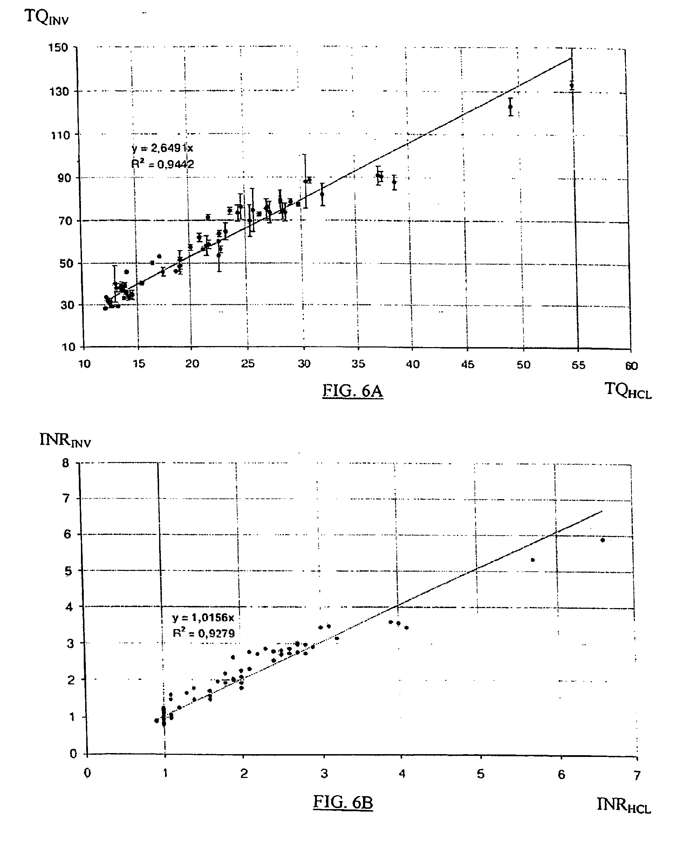

La figure 6A montre la droite de corrélation pour le temps de Quick mesuré par la méthode de l'invention, $TQ_{INV}$ et le même temps mesuré par la méthode de référence $TQ_{HCL}$. On peut vérifier que, si les valeurs absolues des temps ne coïncident pas, elles présentent un très bon coefficient de régression par rapport à la droite de corrélation (coefficient de régression $R^2$ supérieur à 0,94). La corrélation serait encore meilleure si on ne prenait pas en compte les deux échantillons avec $TQ_{HCL}$ > 40 s, qui correspondent à des situations d'urgence médicale dans lesquelles l'utilisation du dispositif et du procédé de l'invention n'est pas prévue.

Comme expliqué plus haut, ce qui intéresse les cliniciens n'est pas tellement le temps de coagulation en tant que tel, mais surtout des paramètres normalisés tels que l'INR défini par la formule suivante :

$$INR=(TQ/TQ_R)^{ISI}$$

où :

- TQ est le temps de coagulation (temps de Quick) de l'échantillon considéré ;
- $TQ_R$ est un temps de référence, correspondant au temps de Quick d'un échantillon de référence ; dans le cas considéré ici, $TQ_R$ =12 s pour la méthode HCL, $TQ_R$ =34 s pour la méthode de l'invention ;
- ISI est un facteur de correction qui dépend des réactifs utilisés pour déclencher la coagulation ; dans le cas considéré ici ISI=1,3 pour les deux méthodes.

La figure 6B montre la droite de corrélation pour l'INR obtenu avec la méthode de référence (INR$_{HCL}$, axe des abscisses) et avec la méthode de l'invention (INR$_{INV}$, axe des ordonnées). On peut voir que la corrélation est très bonne (coefficient de régression = 0,93) et la pente de la droite est proche de 1.

Le procédé de l'invention a été testé avec des échantillons de sang complet, ce qui est avantageux pour les raisons évoquées plus haut. Cependant, se pose le problème de l'influence éventuelle du taux d'hématocrite des échantillons sur le temps de corrélation mesuré.

Pour étudier cet effet, les échantillons de sang entier ont été subdivisés en quatre familles en fonction de leur taux d'hématocrite :

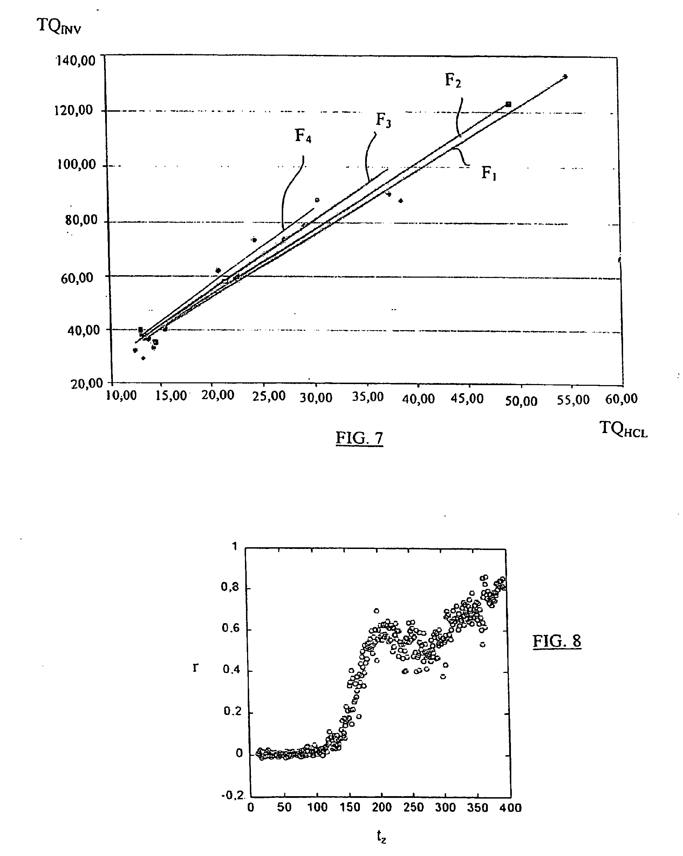

F1 : hématocrite inférieur à 31%
F2 : hématocrite compris entre 31 % et 36%
F3 : hématocrite compris entre 36% et 40%
F4 : hématocrite supérieur à 40 %
et les droites de corrélations TQ$_{INV}$/TQ$_{HCL}$ correspondantes ont été tracées sur la figure 7.

Les pentes des droites de corrélation des différentes familles sont proches, avec un écart maximal de l'ordre de 10%, ce qui confirme qu'il n'y a pas d'influence significative du taux d'hématocrite sur la mesure du temps de coagulation par le procédé de l'invention.

En l'absence de réactif permettant de déclencher de phénomène de coagulation, par exemple lors d'une mesure du temps de coagulation naturelle, il peut se produire un phénomène de sédimentation des cellules du sang sous l'effet de leur propre poids. Cet effet conduit également, comme la coagulation, à une augmentation du coefficient de corrélation entre images de motifs de granularité, comme montré sur la figure 8.

La vitesse de sédimentation est liée à un grand nombre de paramètres du sang tels que le nombre de globules rouges, leur volume, le taux de certaines protéines, la viscosité du sang, .... Ce paramètre n'est pas directement spécifique d'une maladie, mais un élément d'orientation diagnostique.

Comme expliqué plus haut, la coagulation du sang s'accompagne d'une diminution de la transmission optique de l'échantillon, et donc de l'intensité lumineuse moyenne enregistrée par la caméra. Au contraire, la sédimentation n'entraine pas de variation significative de la transmission optique de l'échantillon. Une mesure jointe du coefficient de corrélation et de l'intensité lumineuse moyenne permet donc de différencier les deux phénomènes.

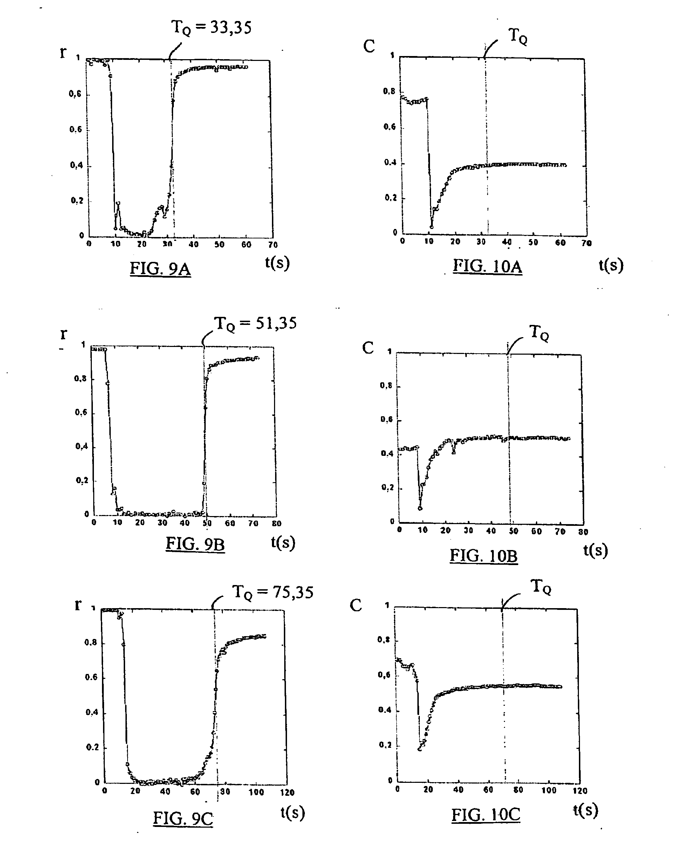

Les figures 9A, 9B et 9C montrent la variation dans le temps du coefficient de corrélation des images de tavelure, mesurée conformément à l'invention, pour trois échantillons de sang entier issus de trois patients différents. Les temps de coagulation TQ mesurés sont respectivement de 33,3s, 51,3s et 75,3 s.

Les figures 10A, 10B et 10C montrent la variation dans le temps du contraste C des images de tavelure. Le contraste est défini comme dans l'article précité de Y. Piederrière.

On peut remarquer que le contraste C change rapidement sur une période beaucoup plus courte que TQ, puis présente une « queue » à variation très lente qui ne permet pas de déterminer de manière précise le temps de coagulation. La comparaison de ces deux lots de figures met donc en évidence l'avantage procuré par le procédé de l'invention.

En particulier lors de mesures permettant la détermination du temps de coagulation naturelle (sans agent de coagulation), on pourra limiter l'effet de sédimentation en plaçant la chambre microfluidique sur un moyen d'agitation, par exemple une platine générant des ondes acoustiques.

L'invention a été décrite plus particulièrement dans son application à l'étude des phénomènes de coagulation et/ou de sédimentation du sang entier. Elle peut néanmoins permettre l'étude des dynamiques de coagulation, sédimentation ou séchage d'autres fluides ou suspensions.

## Revendications

**1.** Procédé de caractérisation de la dynamique de coagulation ou de sédimentation d'un fluide tel que le sang entier, une fraction du sang ou le plasma sanguin, comportant :

- l'éclairage d'un échantillon dudit fluide (ES) avec un faisceau de lumière cohérente ;
- l'acquisition d'une série temporelle d'images d'un motif de granularité engendré par l'interaction entre ledit échantillon et ledit faisceau de lumière spatialement cohérente ; et
- le traitement de ladite série temporelle d'images ; **caractérisé en ce que** ladite étape de traitement comporte :
- le calcul d'une fonction (r) représentative de la variation spatiale dudit motif de granularité entre deux ou plusieurs images de la série ; et
- l'analyse de l'évolution temporelle de ladite fonction.

**2.** Procédé selon la revendication 1, dans lequel ladite étape de traitement comporte la détermination d'un temps de coagulation ou de sédimentation par analyse de l'évolution temporelle de ladite fonction (r) représentative de la variation dudit motif de granularité entre deux ou plusieurs images de la série.

**3.** Procédé selon l'une des revendications précédentes, dans lequel ladite fonction (r) représentative de la variation spatiale dudit motif de granularité entre deux ou plusieurs images de la série est une fonction de corrélation (r) entre paires d'images successives.

**4.** Procédé selon les revendications 2 et 3, dans lequel ladite étape de traitement comporte l'identification d'un point d'inflexion (PI) d'une courbe représentative de l'évolution temporelle de ladite fonction de corrélation.

**5.** Procédé selon l'une des revendications précédentes, comportant également la détermination de la variation temporelle de la transmission optique dudit échantillon, et son utilisation pour discriminer une dynamique de coagulation d'une dynamique de sédimentation.

**6.** Procédé selon la revendication 5, dans lequel la variation temporelle de la transmission optique dudit échantillon est déterminée à partir de ladite série temporelle d'images.

**7.** Procédé selon l'une des revendications précédentes dans lequel lesdites images sont acquises avec une cadence supérieure ou égale à 0,5 Hz.

**8.** Procédé selon l'une des revendications précédentes dans lequel l'échantillon est constitué de sang entier mélangé avec des réactifs, la longueur du chemin optique dudit faisceau de lumière spatialement cohérente à travers ledit échantillon étant comprise entre 20 et 1000 $\mu$m.

**9.** Dispositif pour la mise en oeuvre d'un procédé de caractérisation de la dynamique de coagulation ou de sédimentation d'un fluide selon l'une des revendications précédentes, comportant :

- un dispositif microfluidique (CMF) présentant une veine fluidique susceptible de recevoir un échantillon (ES) dudit fluide et permettant son éclairage ;
- un moyen d'éclairage (L) dudit échantillon par un faisceau de lumière spatialement cohérente ;
- un capteur d'images (C) agencé pour permettre l'acquisition d'une série temporelle d'images d'un motif optique de granularité engendré par l'interaction entre ledit échantillon et ledit faisceau de lumière cohérente ; et
- un moyen de traitement (MT) de ladite série temporelle d'images ;

**caractérisé en ce que** ledit moyen de traitement est adapté pour calculer une fonction représentative de la variation dudit motif de granularité entre deux ou plusieurs images de la série et pour analyser l'évolution temporelle de ladite fonction.

**10.** Dispositif selon la revendication 9 dans lequel ledit capteur d'images est décalé par rapport à la trajectoire directe dudit faisceau de lumière spatialement cohérente de manière à ne pas être ébloui.

**11.** Dispositif selon l'une des revendications 9 et 10 dans lequel ledit capteur d'images est dépourvu de lentilles.

**12.** Dispositif selon l'une des revendications 9 à 11 dans lequel ledit moyen d'éclairage et ledit capteur d'images sont agencés de deux côtés opposés de la veine fluidique, qui est transparente, moyennant quoi lesdites images sont acquises par transmission.

**13.** Dispositif selon la revendication 12 dans lequel ladite veine fluidique a une épaisseur comprise entre 20 et 1000 $\mu$m, et de préférence entre 30 $\mu$m et 300 $\mu$m.

**14.** Dispositif selon l'une des revendications 9 à 11 dans lequel ledit moyen d'éclairage et ledit capteur d'images sont agencés d'un même côté de la veine fluidique, un élément réfléchissant (SR) étant prévu du côté opposé de cette dernière, moyennant quoi lesdites images sont acquises par réflexion.

**15.** Dispositif selon la revendication 14 dans lequel ladite veine fluidique a une épaisseur comprise entre 25 et 500 $\mu$m, et de préférence entre 50 $\mu$m et 150 $\mu$m.

**Claims**

1. A method of characterizing the coagulation or sedimentation dynamics of a fluid such as whole blood, a blood fraction, or blood plasma, the method comprising:

   • illuminating a sample of said fluid (ES) with a beam of coherent light;
   • acquiring a time series of images of a speckle pattern generated by interaction between said sample and said spatially coherent light beam; and
   • processing said time series of images;

   **characterized in that** said processing step includes calculating a function (r) representative of the spatial variation of said speckle pattern between two or more images of the series, and analyzing the temporal evolution of said function.

2. A method according to claim 1, wherein said processing step also includes determining a coagulation or sedimentation time by analyzing the temporal evolution of said function (r) representative of the variation of said speckle pattern between two or more images of the series.

3. A method according to any of the preceding claims, wherein said function (r) representative of the spatial variation of said speckle pattern between two or more images of the series is a correlation function (r) between successive image pairs.

4. A method according to claims 2 and 3, wherein said processing step includes identifying a point of inflection of a curve representative of the temporal evolution of said correlation function.

5. A method according to any of the preceding claims, also including determining the time variation in the optical transmission of said sample, and using said variation to discriminate between coagulation dynamics and sedimentation dynamics.

6. A method according to claim 5, wherein the time variation in the optical transmission of said sample is determined from said time series of images.

7. A method according to any of the preceding claims, wherein said images are acquired at a rate greater than or equal to 0.5 Hz.

8. A method according to any of the preceding claims, wherein the sample is constituted by whole blood mixed with reagents, the optical path length of said spatially coherent light beam through said sample lying in the range 20 $\mu$m to 1000 $\mu$m.

9. A device for implementing a method according to any of the preceding claims for characterizing the coagulation or sedimentation dynamics of a fluid, the device comprising:

   • a microfluidic device (CMF) having a fluid flow passage suitable for receiving a sample (ES) of said fluid and enabling it to be illuminated;
   • lighting means (L) for illuminating said sample by a spatially coherent light beam;
   • an image sensor (C) for acquiring a time series of images of an optical speckle pattern generated by interaction between said sample and said coherent light beam; and
   • a processor means (MT) for processing said time series of images;

   wherein said processor means is adapted to calculate a function representative of the variation of said speckle pattern between two or more images of the series.

10. A device according to claim 9, wherein said image sensor is offset relative to the direct path of said spatially coherent light beam so as to avoid being dazzled.

11. A device according to any of claims 9 and 10, wherein said image sensor is lens-free.

12. A device according to any of claims 9 to 11, wherein said lighting means and said image sensor are arranged on two opposite sides of the fluid flow passage, which passage is transparent, thereby causing said images to be acquired by transmission.

**13.** A device according to claim 12, wherein said fluid flow passage is of thickness lying in the range 20 $\mu$m to 1000 $\mu$m, preferably in the range 30 $\mu$m to 300 $\mu$m.

**14.** A device according to any of claims 9 to 11, wherein said lighting means and said image sensor are arranged on the same side of the fluid flow passage, a reflecting element(SR) being provided on the opposite side therefrom, whereby said images are acquired by reflection.

**15.** A device according to claim 14, wherein said fluid flow passage has thickness lying in the range 25 $\mu$m to 500 $\mu$m, and preferably in the range 50 $\mu$m to 150 $\mu$m.

**Patentansprüche**

**1.** Verfahren zur Charakterisierung der Koagulations- oder Sedimentationsdynamik eines Fluids, wie z. B. Gesamtblut, eine Blutfraktion oder Blutplasma, umfassend:

- Beleuchtung einer Probe des Fluids (ES) mit einem Strahl kohärenten Lichts;
- Erfassung einer zeitlichen Serie von Bildern eines Granularitätsmusters, welches durch die Wechselwirkung zwischen der Probe und dem Strahl räumlich kohärenten Lichts erzeugt wurde; und
- Verarbeitung der zeitlichen Serie von Bildern; **dadurch gekennzeichnet, dass** der Verarbeitungsschritt umfasst:
- Berechnung einer Funktion (r), welche die räumliche Variation des Granularitätsmusters zwischen zwei oder mehreren Bildern der Serie darstellt; und
- Analyse der zeitlichen Entwicklung der Funktion.

**2.** Verfahren nach Anspruch 1, wobei der Verarbeitungsschritt die Bestimmung einer Koagulations- oder Sedimentationszeit durch Analyse der zeitlichen Entwicklung der Funktion (r), welche die Variation des Granularitätsmusters zwischen zwei oder mehreren Bildern der Serie darstellt, umfasst.

**3.** Verfahren nach einem der vorhergehenden Ansprüche, wobei die Funktion (r), welche die räumliche Variation des Granularitätsmusters zwischen zwei oder mehreren Bildern der Serie darstellt, eine Korrelationsfunktion (r) zwischen Paaren von aufeinander folgenden Bildern ist.

**4.** Verfahren nach den Ansprüchen 2 und 3, wobei der Verarbeitungsschritt die Identifikation eines Wendepunkts (PI) einer Kurve, welche die zeitliche Entwicklung der Korrelationsfunktion darstellt, umfasst.

**5.** Verfahren nach einem der vorhergehenden Ansprüche, umfassend außerdem die Bestimmung der zeitlichen Variation der optischen Transmission der Probe und deren Verwendung zur Unterscheidung einer Koagulationsdynamik von einer Sedimentationsdynamik.

**6.** Verfahren nach Anspruch 5, wobei die zeitliche Variation der optischen Transmission der Probe ausgehend von der zeitlichen Serie von Bildern bestimmt wird.

**7.** Verfahren nach einem der vorhergehenden Ansprüche, wobei die Bilder mit einem Takt von größer oder gleich 0,5 Hz erfasst werden.

**8.** Verfahren nach einem der vorhergehenden Ansprüche, wobei die Probe aus mit Reagenzien gemischten Gesamtblut gebildet ist, wobei die Länge des optischen Weges des Strahls räumlich kohärenten Lichts durch die Probe zwischen 20 und 1000 $\mu$m enthalten ist.

**9.** Vorrichtung zum Einsatz eines Verfahrens zur Charakterisierung der Koagulations- oder Sedimentationsdynamik eines Fluids gemäß einem der vorhergehenden Ansprüche, umfassend:

- eine Mikrofluidik-Vorrichtung (CMF), welche eine Fluidader aufweist, die dazu geeignet ist, eine Probe (ES) des Fluids aufzunehmen und ihre Beleuchtung ermöglicht;

- ein Mittel zur Beleuchtung (L) der Probe durch einen Strahl räumlich kohärenten Lichts;
- einen Bildaufnehmer (C), welcher dazu ausgestaltet ist, die Erfassung einer zeitlichen Serie von Bildern eines optischen Granularitätsmusters, welches durch die Wechselwirkung zwischen der Probe und dem Strahl kohärenten Lichts erzeugt wurde, zu ermöglichen; und
- ein Mittel zur Verarbeitung (MT) der zeitlichen Serie von Bildern;

**dadurch gekennzeichnet, dass** das Mittel zur Verarbeitung dazu ausgestaltet ist, eine Funktion zu berechnen, welche die Variation des Granularitätsmusters zwischen zwei oder mehreren Bildern der Serie darstellt, und die zeitliche Entwicklung der Funktion zu analysieren.

10. Vorrichtung nach Anspruch 9, wobei der Bildaufnehmer bezüglich der direkten Trajektorie des Strahls räumlich kohärenten Lichts derart versetzt ist, dass er nicht geblendet wird.

11. Vorrichtung nach einem der Ansprüche 9 und 10, wobei der Bildaufnehmer ohne Linsen ist.

12. Vorrichtung nach einem der Ansprüche 9-11, wobei das Mittel zur Beleuchtung und der Bildaufnehmer auf zwei gegenüberliegenden Seiten der Fluidader, welche transparent ist, angeordnet sind, wodurch die Bilder durch Transmission erfasst werden.

13. Vorrichtung nach Anspruch 12, wobei die Fluidader eine Dicke aufweist, welche zwischen 20 und 1000 $\mu$m, und vorzugsweise zwischen 30 $\mu$m und 300 $\mu$m enthalten ist.

14. Vorrichtung nach einem der Ansprüche 9-11, wobei das Mittel zur Beleuchtung und der Bildaufnehmer auf derselben Seite der Fluidader angeordnet sind, wobei ein reflektierendes Element (SR) auf der gegenüberliegenden Seite der letzteren vorgesehen ist, wodurch die Bilder durch Reflexion erfasst werden.

15. Vorrichtung nach Anspruch 14, wobei die Fluidader eine Dicke aufweist, welche zwischen 25 und 500 $\mu$m und vorzugsweise zwischen 50 $\mu$m und 150 $\mu$m enthalten ist.

FIG.1

ES    CMF

FIG.2A

CMF    S

FIG.2B

MT

C

L

CMF
ES

M

FIG.3A

MT    M    L

C

ES    CMF
SR

FIG.3B

FIG.4A

FIG.4B

r

FIG. 5A          t(s)

$\dfrac{dr}{dt}$

TQ

FIG. 5B          t(s)

FIG. 6A

FIG. 6B

17

FIG. 7

FIG. 8

FIG. 9A

FIG. 10A

FIG. 9B

FIG. 10B

FIG. 9C

FIG. 10C

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4252536 A
- US 3635678 A
- US 6352630 B

**Littérature non-brevet citée dans la description**

- **YANN PIEDERRIÈRE et al.** *Evaluation of blood plasma coagulation dynamics by speckle analysis*